# EUROPEAN PATENT SPECIFICATION

(11) **EP 3 444 285 B1**
(45) Date of publication and mention of the grant of the patent: **22.04.2020**
(21) Application number: 17209113.4
(22) Date of filing: 20.12.2017
(51) Int. Cl.: C08G 12/32, C07D 251/64

(54) **A PROCESS OF CYCLICALLY PRODUCING HEXAMETHYLOLMELAMINE**
ZYKLISCHE HERSTELLUNG VON HEXAMETHYLOLMELAMIN
PRODUCTION CYCLIQUE D'HEXAMÉTHYLOLMÉLAMINE

(30) Priority: 15.08.2017 CN 201710696130
(43) Date of publication of application: 20.02.2019
(73) Proprietor: Li, Ping, 400002 Chongqin Chongqing (CN)
(72) Inventor: LI, Ping, Chongqin, Chongqing 400002 (CN); WANG, Wenwu, Chongqing, Chongqing 400002 (CN); TANG, Xuejun, Chongqing, Chongqing 400002 (CN); TANG, Qiong, Chongqing, Chongqing 400002 (CN)
(74) Representative: Dantz, Jan Henning

(56) References cited:
- EP-A1- 1 607 391
- CN-A- 102 010 380
- CN-A- 102 911 023

## Description

### Field of the Disclosure

The present disclosure relates to a process of cyclically producing hexamethylolmelamine.

### Background of the Disclosure

Hexamethylolmelamine conducts etherification reaction with alcohols in the industry of rubber adhesives to produce a rubber adhesive A, which is extensively applied in increasing adhesion of the rubber with cords. Etherification products may further be applied to coil coating and vehicle coating additives.

At present, hexamethylolmelamine is generally produced by allowing melamine and 37% formaldehyde aqueous solution or paraformaldehyde as raw materials to have hydroxymethylation reaction under an alkaline condition, and then by performing dehydration and drying. A problem with these technologies is that there is so much formaldehyde-containing waste water and thus so much pollution, leaving a mother liquid after separation and dehydration pasty, which does not facilitate recovery of the mother liquid. In addition, the number of methylols is below 5.6, and hard to top 5.8. Besides, a filtration fabric is apt to be obstructed upon centrifugal separation and cannot be cleaned conveniently. A lot of formaldehyde-containing waste water is produced upon cleaning and cannot be effectively recycled or processed.

CN 102010380 A dislcoses a process for producing hexamethylol melamine. The process comprises the steps such as mixing, reaction and crystallization, centrifugalization, evaporation and the like, wherein evaporation auxiliary formate is added when in evaporation so as to change the relative volatility of formaldehyde and water. Furthermore, the process for producing the hexamethylol melamine together with a formaldehyde device can also be used for co-production, so that the concentration of the produced formaldehyde solution is improved to 45wt%-55wt%. The produced hexamethylol melamine provided has a small amount of waste water and low formaldehyde content.

### Summary of the Disclosure

An object of the present disclosure provides a process or method of cyclically producing hexamethylolmelamine with zero-pollution emission.

The process of cyclically producing hexamethylolmelamine according to the present disclosure comprises:
1) adding water or (cyclic) mother liquid in a hydroxylation reaction kettle;
2) then adding formaldehyde aqueous solution in the reaction kettle and keeping content of formaldehyde in a range of 10%-25%;
3) stirring and heating solution in the kettle to 40-80 °C;
4) adding alkali in the reaction kettle, and pre-adjusting a pH value of the solution in the kettle between 8.5-9.5;
5) then adding melamine in the reaction kettle so that a molar ratio of melamine to formaldehyde is 1:6-15;
6) adding alkali again into the reaction kettle, and adjusting the pH value of the solution in the kettle between 8.5-10.0;
7) raising the temperature of the reaction solution in the kettle to 60-85°C, and maintaining the temperature 1-4 hours;
8) introducing the solution in the kettle which has already cooled to below 40°C into a centrifuge for centrifugal separation to obtain hexamethylolmelamine wet material and (cyclic) mother liquid;
9) delivering the hexamethylolmelamine wet material to a drying system for subsequent drying;
10) returning the (cyclic) mother liquid back to the reaction kettle; and
   repeating the above steps 2) to 10) for cyclic production,
   wherein the added formaldehyde aqueous solution is prepared in situ and its concentration is higher than 55%.

In preferred embodiments of the present disclosure, the content of formaldehyde in step 2) is preferably kept at 20%; the temperature in step 3) is preferably 50°C-70°C, more preferably 60°C; the pH value of step 3) is preferably 9.0; the molar ratio in step 5) is preferably 1: 8-10, more preferably 1: 8.5; the pH value in step 6) is preferably 9.0; in step 7), the temperature is preferably 80°C, and the temperature is preferably maintained 3 hours.

The concentration of the high-concentration formaldehyde aqueous solution prepared according to the present disclosure is preferably higher than 55% and is more preferably 58%.

According to an embodiment of the present disclosure, the reaction kettle is disposed on the centrifuge and the mother liquid out of the centrifuge in step 9) is pumped back to the reaction kettle.

According to a preferred embodiment of the present disclosure, a procedure of preparing high-concentration formaldehyde aqueous solution in situ comprises:
introducing methanol and air into an evaporator;
introducing gases from the evaporator and distributed steam together into a superheater;
allowing gases from the superheater through a flame retardant filter;
introducing gases from the flame retardant filter into an oxidizer;
introducing formaldehyde gas from the oxidizer into a first spray tower;
introducing formaldehyde gas from the first spray tower into a second spray tower;
introducing formaldehyde gas from the second spray tower into a third spray tower; and
introducing formaldehyde exhaust gas from the third spray tower into an exhaust gas boiler,
wherein spraying water is introduced from the external into a tower top of the third spray tower, then gas-absorbed water from a tower bottom of the third spray tower is introduced into a tower top of the second spray tower, and gas-absorbed water from a tower bottom of the second spray tower is introduced into a tower top of the first spray tower,
gas-absorbed water at a tower bottom of the first spray tower is cyclically pumped to the tower top of the first spray tower for use as cyclic spray water until the cyclic spray water, after reaching a predetermined formaldehyde solution concentration, is discharged,
the gas-absorbed water at the tower bottom of the second spray tower is pumped to the tower top of the second spray tower for use as cyclic spray water until the cyclic spray water, after undergoing circulation of multiple times, is pumped into the tower top of the first spray tower,
the gas-absorbed water at the tower bottom of the third spray tower is pumped to the tower top of the third spray tower for use as cyclic spray water until the cyclic spray water, after undergoing circulation of multiple times, is discharged into the tower top of the second spray tower.

According to a preferred embodiment of the present disclosure, heat generated by the exhaust gas boiler is used to prepare distributed steam.

The production process according to the present disclosure, particularly in-situ preparation and use of high-concentration formaldehyde, enables the mother liquid to be cyclically used for a production procedure, thereby substantially reducing the product costs and achieving zero-pollution emission.

### Brief Description of Drawings

Fig. 1 is a schematic diagram of a flow chart of a process of producing hexamethylolmelamine according to the present disclosure; and
Fig. 2 is a schematic diagram of a flow chart of a process of preparing high-concentration formaldehyde aqueous solution in situ according to the present disclosure.

### Detailed Description of Preferred Embodiments

The present disclosure will be described in detail with reference to figures.

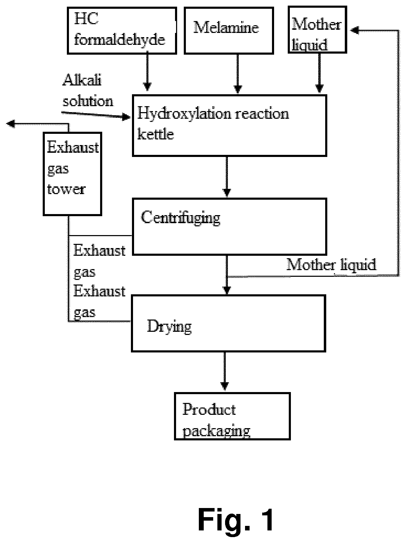

As shown in Fig. 1, a process of producing hexamethylolmelamine according to the present disclosure generally comprises adding formaldehyde, melamine and alkali in a hydroxylation reaction kettle for reaction, performing centrifugation and drying to obtain the product and then conducting packaging. Exhaust gas generated during centrifugation and drying is introduced to an exhaust gas processing tower for subsequent processing. A mother liquid from centrifugal separation is cyclically used as a production raw material in the whole procedure. A specific cyclic production process is as follows:
1) adding water or (cyclic) mother liquid in the hydroxylation reaction kettle;
2) then adding formaldehyde aqueous solution in the reaction kettle and keeping content of formaldehyde in a range of 10%-25%, preferably 15%-20%;
3) stirring and heating solution in the kettle to 40-80°C, preferably 50°C-70°C, more preferably 60°C: in the range of temperature the formaldehyde cannot be excessively volatile while the melamine can dissolve quickly;
4) adding alkali for example sodium hydroxide solution in the reaction kettle, and pre-adjusting pH value of the solution in the kettle between 8.5-9.5, preferably about 9.0: pre-adjusting the pH value is very crucial, and it may accurately control a reaction chemical stoichiometric ratio and a reaction speed in subsequent steps. In addition, the pH value selected at about 9.0 may very well adapt for a fall of the pH value when melamine is added subsequently, so that the amount of the alkali fed upon secondary adjustment is very tiny to facilitate regulation;
5) then adding melamine in the reaction kettle so that a molar ratio of melamine to formaldehyde is 1:6-15, preferably 1: 8-10, more preferably 1: 8.5;
6) adding alkali (sodium hydroxide solution) again into the reaction kettle, and adjusting the pH value of the solution in the kettle between 8.5-10.0, preferably 9.0; the pH value resulting from the secondary adjustment remains substantially consistent with the pH value resulting from the pre-adjustment;
7) raising the temperature of the reaction solution in the kettle to 60-85 °C, for example 80°C, and maintaining the temperature 1-4 hours, for example three hours;
8) introducing the solution in the kettle which has already cooled to below 40°C into a centrifuge for centrifugal separation to obtain hexamethylolmelamine wet material and (cyclic) mother liquid: the reaction kettle may be disposed on the centrifuge so that a reaction product automatically flows into the centrifuge due to gravity for separation, and the mother liquid after separation may be pumped back to the reaction kettle;
9) delivering the hexamethylolmelamine wet material to a drying system for subsequent drying (and packaging);
10) pumping the (cyclic) mother liquid back to the reaction kettle; and
   repeating the above steps 2) to 10) for cyclic production.

According to the present disclosure, during the aforesaid cyclic production, the added formaldehyde aqueous solution is prepared in situ and its concentration is higher than 55%, more preferably 56%-60%, and most preferably 58%. In-situ preparation is performed through a full automatic production line, and the prepared high-concentration formaldehyde is replenished on the spot to participate the aforesaid cyclic production. Use of the high-concentration formaldehyde enables chemical stoichiometric ratios of materials during the above cyclic production process in the reaction kettle of up to 10 cubic meters to still stably remain in a predetermined range and achieves the possibility of sufficiently using the mother liquid to industrialize cyclic production of hexamethylolmelamine.

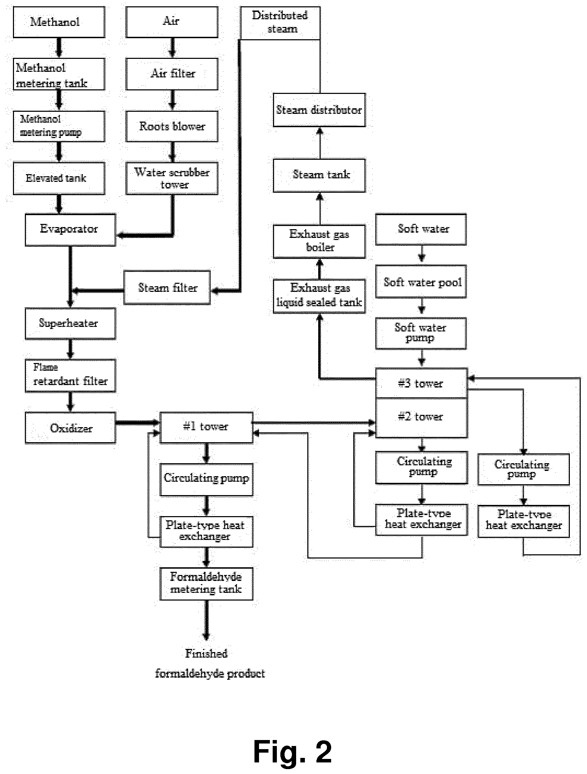

Fig. 2 is a schematic diagram of preparing high-concentration formaldehyde according to the present disclosure. As shown in Fig. 2, the process of preparing high-concentration formaldehyde in situ according to the present disclosure comprises:
delivering methanol from a metering tank to an elevated tank through a metering pump, and allowing methanol to flow from the elevated tank to an evaporator;
in addition, air passes through an air filter, then through a Roots blower, and then through a water scrubber and then into the evaporator, and a temperature of the evaporator is set between 60°C and 70 °C;
on the one hand, the gases out of the evaporator is introduced into a superheater, and on the other hand, distributed steam passes through a steam filter and then into the superheater, and a temperature of the superheater is set between 110°C and 120°C;
mixed gases out of the superheat passes through a flame retardant filter, and then enters an oxidizer to conduct silver catalytic reaction to generate a formaldehyde gas;
introducing the formaldehyde gas from the oxidizer into a first spray tower;
introducing the formaldehyde gas from the first spray tower into a second spray tower;
introducing the formaldehyde gas from the second spray tower into a third spray tower; and
introducing the formaldehyde exhaust gas from the third spray tower into an exhaust gas boiler.
introducing spraying water from the external into a tower top of the third spray tower, wherein the spraying water is soft water pumped from a soft water pool; introducing gas-absorbed water from a tower bottom of the third spray tower into a tower top of the second spray tower; and introducing gas-absorbed water from a tower bottom of the second spray tower into a tower top of the first spray tower.

The second spray tower is directly disposed under the third spray tower so that the gas-absorbed water at the tower bottom of the third spray tower can automatically flow into the tower top of the second spray tower due to gravity. This arrangement is compact and can save water pumps.

The gas-absorbed water at the tower bottom of the first spray tower is cyclically pumped to the tower top of the first spray tower for use as cyclic spray water until the cyclic spray water, after being detected as having reached a predetermined formaldehyde solution concentration, is led out to the formaldehyde metering tank and becomes the high-concentration formaldehyde used in the present disclosure.

The gas-absorbed water at the tower bottom of the second spray tower is pumped to the tower top of the second spray tower for use as cyclic spray water until the cyclic spray water, after undergoing circulation of several times for example three times, is pumped into the tower top of the first spray tower.

The gas-absorbed water at the tower bottom of the third spray tower is pumped to the tower top of the third spray tower for use as cyclic spray water until the cyclic spray water, after undergoing circulation of several times for example three times, is discharged into the tower top of the second spray tower.

The respective spray towers are each mounted with a circulating pump, and additionally provided with a plate-type heat exchanger to reduce the formaldehyde gas temperature and recover heat.

Fig. 2 further shows using heat generated by the exhaust gas boiler to provide distributed steam through a gas tank and a steam distributor.

In the present disclosure, three stages of absorbing towers are used to gradually absorb formaldehyde gas and improve formaldehyde concentration stage by stage, thereby providing up to 60% formaldehyde solution and breaking an upper limit of the concentration of normal formaldehyde solutions, so that cyclic production of hexamethylolmelamine of the present disclosure is implemented in an industrialized manner, and thereby the production costs are substantially reduced and zero-pollution emission is achieved.

Those skilled in the art should appreciate that the above depictions are only intended to facilitate better understanding of the present disclosure, not to limit the present disclosure in any way.

## Claims

1. A process of cyclically producing hexamethylolmelamine, comprising:
1) adding water or mother liquid in a hydroxylation reaction kettle;
2) then adding formaldehyde aqueous solution in the reaction kettle and keeping content of formaldehyde in a range of 10%-25%;
3) stirring and heating the solution in the kettle to 40-80°C;
4) adding alkali in the reaction kettle, and pre-adjusting a pH value of the solution in the kettle between 8.5-9.5;
5) then adding melamine in the reaction kettle so that a molar ratio of melamine to formaldehyde is 1:6-15;
6) adding alkali again into the reaction kettle, and adjusting the pH value of the solution in the kettle between 8.5-10.0;
7) raising a temperature of the reaction solution in the kettle to 60-85 °C, and maintaining the temperature 1-4 hours;
8) introducing the solution in the kettle which has already cooled to below 40°C into a centrifuge for centrifugal separation to obtain hexamethylolmelamine wet material and the mother liquid;
9) delivering the hexamethylolmelamine wet material to a drying system for subsequent drying;
10) returning the mother liquid back to the reaction kettle; and
repeating the above steps 2) to 10) for cyclic production,
wherein the added formaldehyde aqueous solution is prepared in situ and its concentration is higher than 55%.

2. The cyclic production according to claim 1, wherein the reaction kettle is disposed on the centrifuge and the mother liquid out of the centrifuge in step 8) is pumped back to the reaction kettle.

3. The cyclic production according to claim 1, wherein a procedure of preparing the formaldehyde aqueous solution in situ comprises:
introducing methanol and air into an evaporator;
introducing gases from the evaporator and distributed steam together into a superheater;
allowing gases from the superheater through a flame retardant filter;
introducing gases from the flame retardant filter into an oxidizer;
introducing formaldehyde gas from the oxidizer into a first spray tower;
introducing formaldehyde gas from the first spray tower into a second spray tower;
introducing formaldehyde gas from the second spray tower into a third spray tower; and
introducing formaldehyde exhaust gas from the third spray tower into an exhaust gas boiler,
wherein spraying water is introduced from the external into a tower top of the third spray tower, then gas-absorbed water from a tower bottom of the third spray tower is introduced into a tower top of the second spray tower, and gas-absorbed water from a tower bottom of the second spray tower is introduced into a tower top of the first spray tower,
gas-absorbed water at a tower bottom of the first spray tower is cyclically pumped to the tower top of the first spray tower for use as cyclic spray water until the cyclic spray water, after reaching a predetermined formaldehyde solution concentration, is discharged,
the gas-absorbed water at the tower bottom of the second spray tower is pumped to the tower top of the second spray tower for use as cyclic spray water until the cyclic spray water, after undergoing circulation of multiple times, is pumped into the tower top of the first spray tower,
the gas-absorbed water at the tower bottom of the third spray tower is pumped to the tower top of the third spray tower for use as cyclic spray water until the cyclic spray water, after undergoing circulation of multiple times, is discharged into the tower top of the second spray tower.

4. The cyclic production according to claim 3, wherein heat generated by the exhaust gas boiler is used to prepare distributed steam.

## Patentansprüche

1. Verfahren zur zyklischen Produktion von Hexamethylolmelamin, umfassend:
1) Zugeben von Wasser oder Mutterlauge in einen Hydroxylierungsreaktionskessel;
2) anschließend Zugeben einer wässrigen Formaldehydlösung in den Reaktionskessel und Halten des Formaldehydgehalts in einem Bereich von 10 % bis 25 %;
3) Rühren und Erhitzen der Lösung in dem Kessel auf 40 bis 80 °C;
4) Zugeben eines Alkalis in den Reaktionskessel und Voreinstellen eines pH-Werts der Lösung in dem Kessel auf 8,5 bis 9,5;
5) anschließend Zugeben von Melamin in den Reaktionskessel, sodass ein Molverhältnis von Melamin zu Formaldehyd 1 : 6 bis 15 ist;
6) neuerliches Zugeben eines Alkalis in den Reaktionskessel und Einstellen des pH-Werts der Lösung in dem Kessel auf 8,5 bis 10,0;
7) Erhöhen einer Temperatur der Reaktionslösung in dem Kessel auf 60 bis 85 °C und Halten der Temperatur während 1 bis 4 Stunden;
8) Einführen der bereits auf unter 40 °C abgekühlten Lösung in dem Kessel in eine Zentrifuge zur zentrifugalen Trennung, um Hexamethylolmelamin-Nassmaterial und die Mutterlauge zu erhalten;
9) Zuführen des Hexamethylolmelamin-Nassmaterials zu einem Trocknungssystem zur nachfolgenden Trocknung;
10) Zurückführen der Mutterlauge zum Reaktionskessel; und
Wiederholen der obigen Schritte 2) bis 10) zur zyklischen Produktion,
wobei die zugegebene wässrige Formaldehydlösung in situ hergestellt wird und ihre Konzentration höher als 55 % ist.

2. Zyklische Produktion nach Anspruch 1, wobei der Reaktionskessel an der Zentrifuge angeordnet ist und die in Schritt 8) aus der Zentrifuge kommende Mutterlauge zum Reaktionskessel zurückgepumpt wird.

3. Zyklische Produktion nach Anspruch 1, wobei ein Vorgang der Herstellung der wässrigen Formaldehydlösung in situ Folgendes umfasst:
Einführen von Methanol und Luft in einen Verdampfer;
gemeinsames Einführen von Gasen aus dem Verdampfer und verteiltem Dampf in einen Überhitzer;
Leiten der Gase aus dem Überhitzer durch einen flammhemmenden Filter;
Einführen der Gase aus dem flammhemmenden Filter in einen Oxidator;
Einführen von Formaldehydgas aus dem Oxidator in einen ersten Sprühturm;
Einführen von Formaldehydgas aus dem ersten Sprühturm in einen zweiten Sprühturm;
Einführen von Formaldehydgas aus dem zweiten Sprühturm in einen dritten Sprühturm; und
Einführen von Formaldehyd-Abgas aus dem dritten Sprühturm in einen Abgasboiler,
wobei Sprühwasser von außen in eine Turmoberseite des dritten Sprühturms eingeführt wird, anschließend Wasser mit absorbiertem Gas von einer Turmunterseite des dritten Sprühturms in eine Turmoberseite des zweiten Sprühturms eingeführt wird und Wasser mit absorbiertem Gas von einer Turmunterseite des zweiten Sprühturms in eine Turmoberseite des ersten Sprühturms eingeführt wird,
wobei Wasser mit absorbiertem Gas an einer Turmunterseite des ersten Sprühturms zyklisch zur Turmoberseite des ersten Sprühturms gepumpt wird, um als zyklisches Sprühwasser verwendet zu werden, bis das zyklische Sprühwasser nach dem Erreichen einer vorbestimmten Konzentration der Formaldehydlösung abgelassen wird,
wobei das Wasser mit absorbiertem Gas an der Turmunterseite des zweiten Sprühturms zur Turmoberseite des zweiten Sprühturms gepumpt wird, um als zyklisches Sprühwasser verwendet zu werden, bis das zyklische Sprühwasser, nachdem es einer mehrmaligen Umwälzung unterzogen wurde, zur Turmoberseite des ersten Sprühturms gepumpt wird,
wobei das Wasser mit absorbiertem Gas an der Turmunterseite des dritten Sprühturms zur Turmoberseite des dritten Sprühturms gepumpt wird, um als zyklisches Sprühwasser verwendet zu werden, bis das zyklische Sprühwasser, nachdem es einer mehrmaligen Umwälzung unterzogen wurde, zur Turmoberseite des zweiten Sprühturms abgelassen wird.

4. Zyklische Produktion nach Anspruch 3, wobei Hitze, die durch den Abgasboiler erzeugt wird, verwendet wird, um verteilten Dampf herzustellen.

## Revendications

1. Procédé de production cyclique d'hexaméthylolmélamine, comprenant :
1) l'ajout d'eau ou d'une solution mère dans un réacteur d'hydroxylation ;
2) puis l'ajout d'une solution aqueuse de formaldéhyde dans le réacteur et la conservation d'une teneur en formaldéhyde dans une plage de 10 % à 25 % ;
3) le brassage et le chauffage de la solution dans le réacteur à 40 à 80 °C ;
4) l'ajout d'alcali dans le réacteur, et le préajustement d'une valeur de pH de la solution dans le réacteur entre 8,5 et 9,5 ;
5) puis l'ajout de mélamine dans le réacteur de sorte qu'un rapport molaire entre mélamine et formaldéhyde soit de 1 : 6 à 15 ;
6) l'ajout à nouveau d'alcali dans le réacteur, et l'ajustement de la valeur de pH de la solution dans le réacteur entre 8,5 et 10 ;
7) l'augmentation d'une température de la solution de réaction dans le réacteur à 60 à 85 °C, et le maintien de la température de 1 à 4 heures ;
8) l'introduction de la solution dans le réacteur qui a déjà été refroidi à moins de 40 °C dans une centrifugeuse pour une séparation centrifuge pour obtenir une matière humide d'hexaméthylomélamine et la solution mère ;
9) l'apport de la matière humide d'hexaméthylomélamine à un système de séchage pour un séchage ultérieur ;
10) le renvoi de la solution mère de retour vers le réacteur ; et
la répétition des étapes 2) à 10) ci-dessus pour une production cyclique,
dans laquelle la solution aqueuse de formaldéhyde ajoutée est préparée *in situ* et sa concentration est supérieure à 55 %.

2. Production cyclique selon la revendication 1, dans laquelle le réacteur est disposé sur la centrifugeuse et la solution mère sortant de la centrifugeuse à l'étape 8) est pompée de retour vers le réacteur.

3. Production cyclique selon la revendication 1, dans laquelle une procédure de préparation de la solution aqueuse de formaldéhyde *in situ* comprend :
l'introduction de méthanol et d'air dans un évaporateur ;
l'introduction de gaz depuis l'évaporateur et de vapeur distribuée ensemble dans un surchauffeur ;
l'admission de gaz depuis le surchauffeur à travers un filtre retardateur de flamme ;
l'introduction de gaz depuis le filtre retardateur de flamme dans un oxydant ;
l'introduction de gaz de formaldéhyde depuis l'oxydant dans une première tour de pulvérisation ;
l'introduction de gaz de formaldéhyde depuis la première tour de pulvérisation dans une deuxième tour de pulvérisation ;
l'introduction de gaz de formaldéhyde depuis la deuxième tour de pulvérisation dans une troisième tour de pulvérisation ; et
l'introduction de gaz effluent de formaldéhyde depuis la troisième tour de pulvérisation dans une chaudière récupératrice,
dans laquelle de l'eau de pulvérisation est introduite depuis l'extérieur dans un dessus de tour de la troisième tour de pulvérisation, puis de l'eau à absorption de gaz depuis un dessous de tour de la troisième tour de pulvérisation est introduite dans un dessus de tour de la deuxième tour de pulvérisation, et de l'eau à absorption de gaz depuis un dessus de tour de la deuxième tour de pulvérisation est introduite dans un dessus de tour de la première tour de pulvérisation,
de l'eau à absorption de gaz au niveau d'un dessous de tour de la première tour de pulvérisation est pompée cycliquement vers le dessus de tour de la première tour de pulvérisation pour son utilisation en tant qu'eau de pulvérisation cyclique jusqu'à ce que l'eau de pulvérisation cyclique, après avoir atteint une concentration en solution de formaldéhyde prédéterminée, soit évacuée,
l'eau à absorption de gaz au niveau du dessous de tour de la deuxième tour de pulvérisation est pompée vers le dessus de tour de la deuxième tour de pulvérisation pour son utilisation en tant qu'eau de pulvérisation cyclique jusqu'à ce que l'eau de pulvérisation cyclique, après avoir subi une circulation de multiples fois, soit pompée dans le dessus de tour de la première tour de pulvérisation,
l'eau à absorption de gaz au niveau du dessous de tour de la troisième tour de pulvérisation est pompée vers le dessus de tour de la troisième tour de pulvérisation pour son utilisation en tant qu'eau de pulvérisation cyclique jusqu'à ce que l'eau de pulvérisation cyclique, après avoir subi une circulation de multiples fois, soit évacuée dans le dessus de tour de la deuxième tour de pulvérisation.

4. Production cyclique selon la revendication 3, dans laquelle la chaleur générée dans la chaudière récupératrice est utilisée pour préparer de la vapeur distribuée.
